# EUROPEAN PATENT SPECIFICATION

(11) **EP 3 476 440 B1**
(45) Date of publication and mention of the grant of the patent: **13.10.2021**
(21) Application number: 18202303.6
(22) Date of filing: 24.10.2018
(51) Int. Cl.: A63B 22/00, A63B 22/02, A63B 24/00

(54) **METHOD AND SYSTEM FOR MANAGING A TRAINING OF USERS ON A PLURALITY OF EXERCISE MACHINES**
VERFAHREN UND SYSTEM ZUR VERWALTUNG EINES TRAININGS VON BENUTZERN AUF MEHREREN ÜBUNGSMASCHINEN
PROCÉDÉ ET SYSTÈME POUR GÉRER UN APPRENTISSAGE D'UTILISATEURS SUR UNE PLURALITÉ D'APPAREILS D'EXERCICE

(30) Priority: 25.10.2017 IT 201700121366
(43) Date of publication of application: 01.05.2019
(73) Proprietor: Technogym S.p.A., 47521 Cesena, Forli'-Cesena (IT)
(72) Inventor: TANA, Marcello, 47521 Cesena, FORLI'-CESENA (IT); MUSSONI, Paola, 47521 Cesena, FORLI'-CESENA (IT); FABBRI, Mauro, 47521 Cesena, FORLI'-CESENA (IT)
(74) Representative: Mozzi, Matteo

(56) References cited:
- US-A1- 2008 207 401
- US-A1- 2015 273 272
- US-A1- 2015 343 250
- US-B1- 6 458 060

## Description

### Field of the invention

The present invention relates to the field of fitness and in particular to a method and system for managing a training of users on a plurality of exercise machines.

### Background

Nowadays, the concept of training class, i.e. training of multiple users each on an exercise machine of a plurality of exercise machines of the same type (e.g. treadmills) arranged inside the same location (gym or club) is very widespread.

A training class is managed and coordinated by a coach or trainer who, in addition to motivating and encouraging users during the training, imparts indications to the user of when and how to change one or more parameters of the specific type of exercise machine, so they can follow and respect a specific training program previously chosen by the trainer for that training class.

For example, if treadmills are used in the training class, the trainer instructs the users to periodically vary the rotation speed and/or the gradient of the treadmill.

When instructed by the trainer, each user modifies the speed and/or the gradient of the treadmill by means of the appropriate control console with which the treadmill is provided.

Such training method is not free from faults.

Indeed, since the trainer's instructions are imparted by voice, some users may not hear the instruction at all if they are concentrated in the physical effort or because of the music or in general because of the background noise present inside the gym or club or may understand a different meaning.

Furthermore, each user has a reaction time regretfully different from that of another person, and so a given instruction may not be imparted to all treadmills at the same time or may not be imparted correctly once it has been received by the user.

So, the training class is not always coordinated and synchronized with respect to the training program previously prepared by the trainer for the training class.

Furthermore, this obviously implies an evident disadvantage for users who may find themselves in situations of breathlessness due to excessive effort with the risk of physical problems, falling from the treadmill and so forth.

Additionally, even the trainers, if they fail to notice that one or more users are struggling on their respective treadmills, would not be able to manage the issue in a timely manner on each treadmill because they would inevitably need to go from one treadmill to the other to go to correct the incorrect commands introduced by users.

US 6,458,060 B1 discloses an exercise device configured to enable a user to interact with a trainer in real-time communication.

US 2015/343250 A1 discloses a multi-axis adjustable exercise machine which is pivotable about both a pitch axis and a roll axis with respect to a base for allowing an exerciser to perform a wide range of exercises on pitched or rolled exercise machine.

US 2008/207401 A1 discloses a group exercise system including a master exercise device including a variable parameter and being operatively associated with a control system and a servant exercise device including the variable parameter and being in communication with the control system.

### Summary

It is the object of the present invention to devise and provide a method and system for managing a training for users on a plurality of exercise machines which allows to solve at least partially the drawbacks illustrated above with reference to the prior art, which is as reliable and safe for users and the trainer as possible.

Such object is achieved by a method according to claim 1.

A system configured to execute the aforesaid method is a further object of the present invention.

### Figures

Further features and advantages of the method and system according to the invention will be apparent from the following description of preferred embodiments, given by way of indicative, non-limiting examples, with reference to the accompanying figures, in which:
- figure 1 shows, by means of a block chart, a system for managing a training of users on a plurality of exercise machines according to an embodiment of the present invention;
- figures 2 and 3 show, by means of a block chart, a component of the system in figure 1, respectively, according to an embodiment of the present invention;
- figures 4, 5 and 6 schematically illustrate respective screens shown by components of the system in figure 1 during their operation, and
- figure 7 shows, by means of a block chart, a method for managing a training of users on a plurality of exercise machines according to an embodiment of the present invention.

### Detailed description

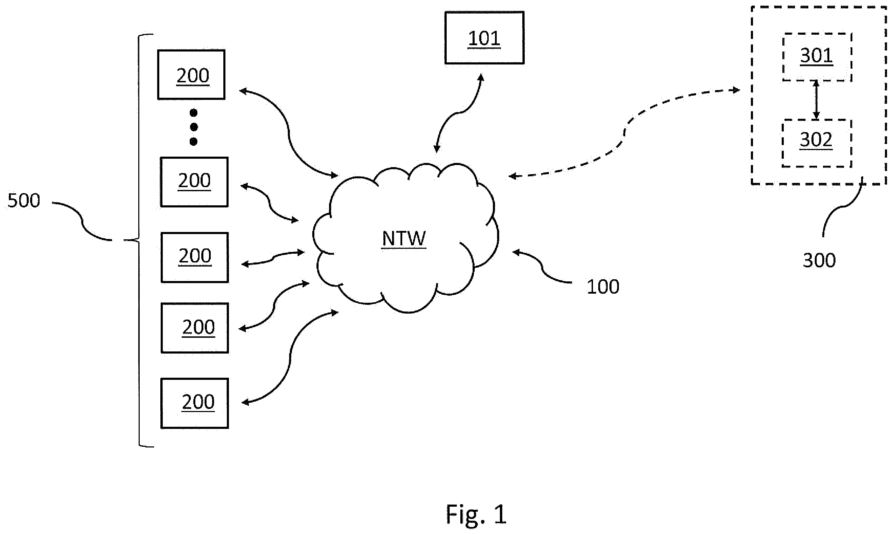

A system 100 for managing a training of users on a plurality of exercise machines, hereinafter also referred to as training system or simply system, according to an embodiment of the present invention, will now be described with reference to figure 1.

It is worth noting that equivalent or similar elements are indicated by the same numerical and/or alphanumerical reference in the aforesaid figures.

The system 100 comprises a control unit 101 of a training of users on a plurality of exercise machines.

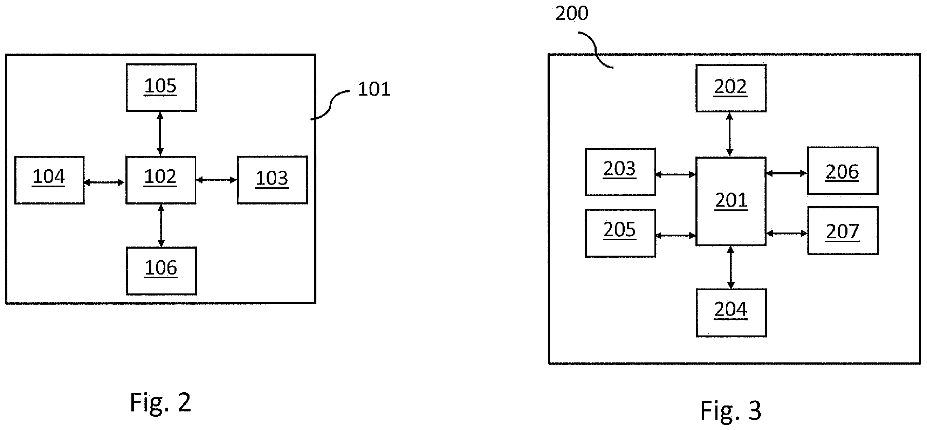

In greater detail, with reference to also figure 2, the control unit 101 comprises a data processing module 102, e.g. a microcontroller or a microprocessor.

The control unit 101 further comprises a memory module 103, operatively connected to the data processing module 102.

The memory module 103 may be either internal or external (as shown in the figure 1, for example) to the data processing module 102.

It is worth noting that the memory module 103 is configured to store one or more program codes which can be executed by the data processing module 102 and data generated and processed following the execution of said one or more program codes.

The data processing module 102 is configured to execute a method for managing the training of users on a plurality of exercise machines according to the present invention, as will be described below.

In this regard, the control unit 101 also comprises a control interface 104, operatively connected to the data processing module 102, configured to allow a trainer user or personal trainer to interact with the control unit 101.

In an embodiment, the control interface 104 may be of the touchscreen type.

In an embodiment alternative to the previous one, the control interface 104 may be a mechanical keyboard.

The control unit 101 further comprises a display module 105, operatively connected to the data processing module 102.

The display module 105 can be used by the trainer user during the interaction with the control unit 101.

In an embodiment, the display module 105 is separate from the control interface 104.

In an embodiment, in which the control interface 104 is of the touchscreen type, the display module 105 coincides with the control interface 104.

Turning back to the embodiment in figure 2, the control unit 101 also comprises a data communication module 106, operatively connected to the data processing module 102, configured to allow the control unit 101 to transmit and receive data.

The functions of the control unit 101, in particular for managing the method for managing a training of users on a plurality of exercise machines, will be described below.

Tuning back in general to figure 1, the system 100 comprises a plurality of exercise machines 500 operatively connected to the control unit 101 by means of a data communication network NTW, i.e. a data communication network of the LAN type or a data communication network with Wi-Fi technology present in a gym or a club.

The plurality of exercise machines 500 are distributed in the same location, such as for example a gym or a club, so that they can be used by the users to perform a so-called training class under the supervision of a coach or trainer user suitable for using use the control unit 101.

For the purposes of the present invention, "exercise machine" means any exercise apparatus which can be used in a training class, such as, for example, a treadmill, a bike, a cyclette, a rower, a spinning machine, a muscle development machine and so on.

First of all, it is worth noting that the plurality of machines 500 and the control unit 101 are configured to communicate with one another by means of the communication network data NTW, following an initial configuration in which the control unit 101 and the plurality of exercise machines 500 are associated with the set training class.

In this regard, in greater detail, each exercise machine of the plurality of exercise machines 500 is configured to store a network address, e.g. an IP address, of the control unit 101.

Hereinafter, with particular reference also to figure 3, an exercise machine 200 belonging to the plurality of exercise machines 500 will be described.

It is worth noting that each exercise machine belonging to the plurality of exercise machines 500 is also indicated by the reference numeral 200 in figure 1.

The following description applies to any exercise machine 200 of the plurality of exercise machines 500 shown in figure 1.

With reference to figure 3, the exercise machine 200 comprises a respective data processing unit 201.

The exercise machine 200 further comprises a memory unit 202, operatively connected to the data processing unit 201.

The memory unit 202 can be either internal or external (as shown in the figure 1, for example) to the data processing unit 201.

It is worth noting that the memory unit 202 is configured to store one or more program codes which can be executed by the data processing unit 201 and data generated and processed following the execution of one or more program codes.

The data processing unit 201 can be configured to control the operation of the exercise machine 200 during the execution of the method for managing the training of users on a plurality of exercise machines according to the present invention, as will be described below.

It is worth noting that the functions of the exercise machine 200, in particular for implementing the method for managing a training of users on a plurality of exercise machines 500, will be described below.

In this respect, for the purposes of the present invention, it is worth noting that "controlling the operation of an exercise machine" means sending of at least one control instruction to the exercise machine during its operation.

"Control instruction of the exercise machine during its operation" means either the instructions sent by the control unit 101 upon command of the trainer user, e.g. a stop command, a pause command, a restart command, an end of training command, and instructions sent automatically by the control unit, e.g. such as a synchronization command, a command to vary one or more parameters of the exercise machine which can be controlled during its operation, such as, for example, the rotation speed in a treadmill, the resistance in a bike or cyclette, the gradient or inclination, e.g. in a treadmill, the level of difficulty, e.g. in a treadmill, in a bike or cyclette or in a muscular strengthening machine and so on.

Turning back to figure 3, the exercise machine 200 further comprises a respective control interface 203, operatively connected to the data processing unit 201, configured to allow a user to interact with the exercise machine.

In an embodiment, the control interface 203 may be of the touchscreen type.

In an embodiment alternative to the previous one, the control interface 203 may be a mechanical keyboard.

The exercise machine 200 further comprises a respective display unit 204 operatively connected to the data processing unit 201.

The display unit 204 can be used by a user, who is training in the training class, during the interaction with the exercise machine 200.

In an embodiment, the display unit 204 is separate from the control interface 203.

In an embodiment, in which the control interface 203 is of the touchscreen type, the display unit 204 coincides with the control interface 203.

The exercise machine 200 further comprises a data communication unit 205, operatively connected to the data processing unit 201.

The data communication unit 205 of the exercise machine 200 is configured to allow the connection to the control unit 101 by means of the communication network data NTW.

In an embodiment, the exercise machine 200 is connected to the control unit 101 by means of the communication network data NTW using a data connection in wireless mode.

In a further embodiment, alternative to the previous one, the exercise machine 200 is connected to the control unit 101 by means of the communication network data NTW using a wired data connection.

According to the connection mode (wired or wireless), the data communication module 106 of the control unit 101 and the data communication unit 205 of the exercise machine 200 implement a respective technology suitable for correct data communication.

In this respect, it is worth noting that adequate data transmission security protocols are employed in the data communication data, by means of the communication network data NTW, between the control unit 101 and the exercise machine 200.

Furthermore, it is worth noting that both the data communication module 106 of the control unit 101 and the data communication unit 205 of the exercise machine 200 are also configured to transmit and receive data to and from any other communication networks, if present.

According to an embodiment, shown in figure 3, in combination with any of the previous ones, the exercise machine 200 comprises an actuation device 206, e.g. an electromechanical linear actuator, operatively connected to the data processing unit 201.

The actuation device 206 of the exercise machine 200 is configured to receive a command from the data processing unit 201 to vary a first parameter of the exercise machine which can be controlled during its operation.

The actuation device 206 of the exercise machine 200, if the latter is a treadmill, may receive a command to vary the gradient of the treadmill.

For example, if the actuation device 206 of the exercise machine is an electromechanical linear actuator, the command to vary the gradient of the treadmill, either increasing or decreasing it, by the data processing unit 201, causes a lifting or lowering of the front portion of the running surface of the treadmill by means of a endless screw-helical wheel kinematic system, respectively.

According to an embodiment, shown in figure 3, in combination with any previous one, the exercise machine 200 comprises an electric motor 207 is operatively connected to the data processing unit 201.

The electric motor 207 of the exercise machine 200 is configured to receive from the data processing unit 201 a command to vary a second parameter of the exercise machine 200 which can be controlled during its operation.

For example, if the exercise machines 200 is a treadmill, the electric motor 207 can receive a command to vary the rotation speed of the electric motor 207 in order to either reduce or increase the speed of the treadmill.

Turning back to figure 1, in an embodiment, in combination with any one of those described above and shown in figure 1 by a dashed line, the system 100 further comprises a remote electronic processor 300 (cloud) operatively connected to the data communication network NTW, e.g. by means of the Internet.

In particular, in such case, the control unit 101 and the plurality of exercise machines 500 are operatively connected to one another by means of the communication network data NTW and with the remote electronic processor 300 by means of the Internet.

The remote electronic processor 300 comprises a respective data processing unit 301 and a respective a memory unit 302 operatively connected to the data processing unit 301.

The memory unit 302 of the remote electronic processor 300 is configured to store data representative of a training performed on the plurality of exercise machines 500 by users who created their own account on the remote electronic processor 300.

Furthermore, the memory unit 302 of the remote electronic processor 300 is configured to store a library comprising training programs which can be performed on the plurality of exercise machines 500.

In other words, the memory unit 302 of the remote electronic processor 300 is a remote database.

It is worth noting that the communication data, e.g. by means of the Internet network, between the control unit 101 and the plurality of exercise machines 500 with the remote electronic processor 300 also envisages the use of appropriate data transmission security protocols.

In a further embodiment, alternative to the previous one, not shown in the figures, the system 100 may be free from the remote electronic processor 300.

Turning back now in general to the system 100 in figure 1 and to the component of figure 2, we will describe the functions of the control unit 101.

The control unit 101, and thus the data processing module 102, is configured to load in the respective memory module 103 a training program to be performed for the users of a plurality of exercise machines 500 (training class), chosen by the trainer user from multiple training programs.

In this regard, in an embodiment, the control unit 101 is configured to receive one or more previously stored training programs from the respective memory module 103.

In an alternative embodiment, the control unit 101 is configured to receive one or more training programs from an electronic device of the trainer user (e.g. a personal computer, a laptop, a tablet, a smartphone).

In this case, the training programs were previously created by the trainer user on the electronic device of the trainer user by means of a specific software application (app).

In a further embodiment, the control unit 101 is configured to receive one or more training programs from the remote electronic processor 300, if provided, or other remote database of the gym or club.

It is worth noting that for the purposes of the present description "training program" means a set of instructions comprising the subdivision into successive intervals of time (steps) (e.g. equal to 3 or 4 minutes), either regular or irregular, if the overall training time (e.g. 25, 30, 40 minutes and so forth) and the allocation for each interval of time into which the total training time is divided by a value of one or more parameters of the exercise machine which can be controlled during its operation.

It is worth noting that adjacent time intervals into which the total training time is divided will preferably have different values of said one or more parameters of the exercise machine which can be controlled during its operation.

In an embodiment, the set of instructions which are representative of the training program for each interval of time into which the total training time is divided may comprise a value of a first parameter of the exercise machine 200 which can be controlled during its operation.

In an embodiment, if the exercise machine 200 is a treadmill, the first parameter of the exercise machine which can be controlled during its operation is the gradient (expressed as a percentage) of the treadmill.

In an embodiment, alternative to the previous one, if the exercise machine 200 is a treadmill, the first parameter of the exercise machine which can be controlled during its operation is the rotation speed of the treadmill.

In a further embodiment, in combination with the previous ones, the set of instructions which are representative of the training program for each interval of time into which the total training time is divided may further comprise a value of a second parameter of the exercise machine 200 which can be controlled during its operation.

In an embodiment, if the exercise machine 200 is a treadmill and the first parameter of the exercise machine which can be controlled during its operation is the gradient of the treadmill, the second parameter of the exercise machine which can be controlled during its operation is the rotation speed of the treadmill.

In a further embodiment, alternative to the previous one, if the exercise machine 200 is a treadmill and the first parameter of the exercise machine which can be controlled during its operation is the rotation speed of the treadmill, the second parameter of the exercise machine which can be controlled during its operation is the gradient of the treadmill.

In an embodiment, in combination with any one of the previous ones, the control unit 101 is configured to show a start of training command to the trainer user, e.g. on the touchscreen type display module 105 (control interface 104).

After pressing such start of training command, the control unit 101 is configured to send, by means of the communication network data NTW, a start of training command to the plurality of exercise machines 500 of the training class.

According to the invention, the sending of the start of training command by the control unit 101 allows to unlock the plurality of exercise machines 500 which, until the start of training command was received, are locked and ready to be used only in the training class with which they were associated.

In an embodiment, in combination with the previous one, the control unit 101 is configured to send to the plurality of exercise machines 500, by means of the communication network data NTW, at least one control instruction of the operation of each exercise machine of the plurality of exercise machines 500.

Examples of control instructions of the operation of each exercise machine were shown above.

In greater detail, in an embodiment, the control unit 101 is configured to send to the plurality of exercise machines 500 such control instruction of the operation in an exercise machine in response to the reception of a respective request and training data of the user on the exercise machine 200 recorded by the exercise machine 200, received periodically (e.g., once a second) from the exercise machine 200 during its operation.

"Training data" means at least one of or a combination of:
- data representative of the user's physical condition (e.g. heart rate, step rate, stride power, calories burned and so on);
- identification data of the user (e.g. the real name or a nickname);
- data representative of the exercise machine (e.g. if the exercise machine is a treadmill, the rotation speed, the gradient and so on).

By way of example, during its operation, the exercise machine 200 is configured to periodically send (e.g. once a second) a request to receive a control instruction of the operation of the exercise machine 200 and training data of the user on the exercise machine 200.

If the control unit 101, according to the loaded training program, in response to the reception of such request, sends to the exercise machine 200 at least one control instruction of the operation of the exercise machine, the exercise machine 200 will modify its operation as a function of the received instruction.

On the other hand, if the control unit 101, according to the loaded training program, in response to the reception of such request, does not send any control instructions of the operation of the exercise machine to the exercise machine 200, the exercise machine 200 will continue its operation as a function of the received instruction.

It is worth noting that the control instructions of the operation of the exercise machine 200 that the control unit 101 may send to the plurality of exercise machines 500 can be executed automatically by the exercise machine 200 as a function of a respective command of the user trainer imparted to the control unit 101.

In this case, the at least one control instruction of the operation of the exercise machine 200 may be a stop command, a pause command, a command to restart the exercise machine 200.

Furthermore, the control instructions of the operation of the exercise machine 200 that the control unit 101 may send to the plurality of exercise machines 500 may be executed automatically by an exercise machine 200, if the control instruction of the operation of the exercise machine 200 is the variation of a parameter of the exercise machine which can be controlled during its operation (e.g. if the exercise machine is a treadmill, the variation of the gradient and/or of the rotation speed of the treadmill).

If the at least one control instruction of the operation of the exercise machine 200 is a stop command of the exercise machine 200, if the exercise machine 200 is a treadmill, the electric motor 207 is controlled so as to stop the treadmill with a set deceleration ramp and the actuating device 206 is controlled so as to take the gradient of the treadmill to a zero value.

If the at least one control instruction of the operation of the exercise machine 200 is a pause command of the exercise machine 200, if the exercise machine 200 is a treadmill, the electric motor 207 is controlled so as to stop the treadmill with a set deceleration ramp and the actuating device 206 is controlled so that the gradient of the treadmill remains unchanged in the interval of time of the training program in which the stop command of the exercise machine 200 was received.

If the at least one control instruction of the operation of the exercise machine 200 is a stop command of the exercise machine 200, if the exercise machine 200 is a treadmill, the electric motor 207 is controlled so as to be restarted at a given speed (e.g. a set value equal to 9 km/h) with a set acceleration ramp.

If the at least one control instruction of the operation of the exercise machine 200 is an end of training command because the training time has ended or following a manual command by the trainer user, if the exercise machine 200 is a treadmill, the electric motor 207 is controlled so as to stop the treadmill with an established deceleration ramp and the actuating device 206 is controlled so as to take the gradient of the treadmill to a null value.

It is worth noting that if the at least one control instruction of the operation of the exercise machine 200 is a pause command of the exercise machine 200, the control unit 101 can momentarily stop the plurality of exercise machines 500 so as to be able to alternate training on the exercise machine 200, according to the loaded training program (e.g. running on a treadmill), with floor training off the exercise machine (e.g. leaps, sit-ups, push-ups).

In an embodiment, the control unit 101 is configured to provide a piece of information representative of a start of training time (zero time) to the plurality of exercise machines 500, by means of the communication network data NTW.

The control unit 101 is further configured to provide to the plurality of exercise machines 500, by means of the communication network data NTW, preferably at regular time intervals, a piece of information representative of the progressive advancement of the training time so that the synchronization of the training program loaded by the control unit 101 is guaranteed across the plurality of exercise machines 500.

Furthermore, in an embodiment, the control unit 101 is configured to provide, by means of the data communication network NTW, a piece of information representative of the total time of the training program.

In an embodiment, either in combination with or alternatively to the previous ones, once the training program selected by the trainer user for the training class is loaded, before sending the start of training command to the plurality of exercise machines 500 of the training class by means of the communication network data NTW, the control unit 101 is configured to send the training program to the plurality of exercise machines 500, by means of the communication network data NTW.

In an embodiment, as previously mentioned, the control unit 101 is configured to receive training data of the user on the exercise machine 200 from the plurality of exercise machines 500, by means of the communication network data NTW, and to store the data in the memory module 103, while performing the training program.

The control unit 101 is also configured to display to the trainer user, by means of the respective display module 105 with which the control unit 101 is provided, a list of users participating in the training program with which training data of the user on the exercise machine 200 are associated, for each user, and an indication representative of whether a user can follow the training program loaded on the control unit 101 by the trainer user or not.

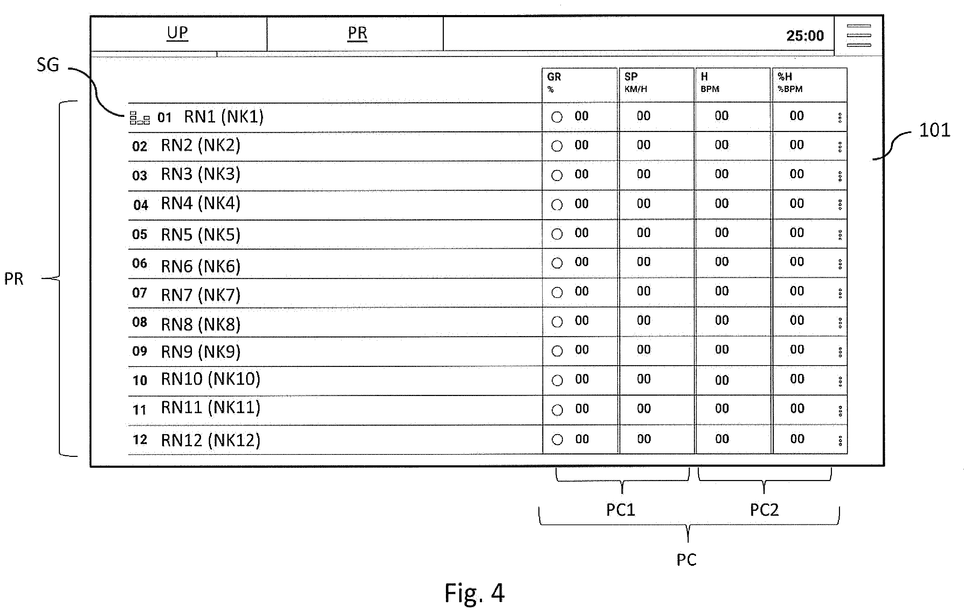

An example of such display is shown in Fig. 4.

The control unit 101 suggests to the trainer user a first screen UP representative of the performance of the users during the execution of the training program.

The first screen UP comprises a plurality of lines PR each reserved for identification data of a user.

Each user is identified by a progressive number (a number from 01 to 12 in the figure), by a respective real name RN1-RN12 and by a respective nickname NK1-NK12.

The first screen UP further comprises a plurality of columns PC in which a first portion PC1 is reserved for data representative of the exercise machine GR, SP (for example, in the case of a treadmill, gradient GR expressed as a percentage and rotation speed SP), while a second portion PC2 is reserved for data representative of the physical condition of the user H, %H (e.g. heart rate and heat rate expressed as a percentage with respect to a set threshold value, as for example the maximum theoretical heart rate, i.e. the mathematical difference between a set value equal to 220 and a value corresponding to the user's age).

It is worth noting that in figure 4, the indication representative of whether a user can follow the training program loaded on the control unit 101 by the trainer user or not materializes in the display of a graphic symbol, indicated in the figure by reference SG, positioned by the side of the progressive number with identifies the user's line.

In particular, if a user, failing to follow the training program loaded on the control unit 101 (and possibly sent to each exercise machine 200), for example, changes the value of the gradient of the treadmill by manually lowering the value with respect to the gradient value automatically controlled by the data processing unit 201 of the exercise machine 200 in the respective interval of time, the control unit 101 is configured to show that user to the trainer user by moving the respective line in the plurality of lines PR, each reserved for identification data of a user, to the first place of said plurality of lines PR, possibly adding the graphic symbol SG alongside the progressive number 01, of the respective real name RN1 and of the respective nickname NK1.

In greater detail, in order to perform the aforesaid movement of a line in the plurality of lines PR, the data processing module 102 is configured to:
- receive a value of said first parameter of the exercise machine 200 which can be controlled during its operation (e.g. the gradient of a treadmill), during the training of a user on the exercise machine 200;
- compare such received value with the value of said first parameter of the exercise machine 200 which can be controlled during its operation envisaged by the training program in order to determine a deviation;
- if such deviation is different from zero, to move the line inside the plurality of lines PR corresponding to such user to the first place of the plurality of lines PR, adding a graphic symbol SG by the side of the progressive number 01, the respective real name RN1 and the respective nickname NK1 of such user.

The control unit 101 is also configured to display to the trainer user, by means of the respective display module 105 with which with the control unit 101 is provided, a training profile representing the training program in progress on the plurality of exercise machines 500.

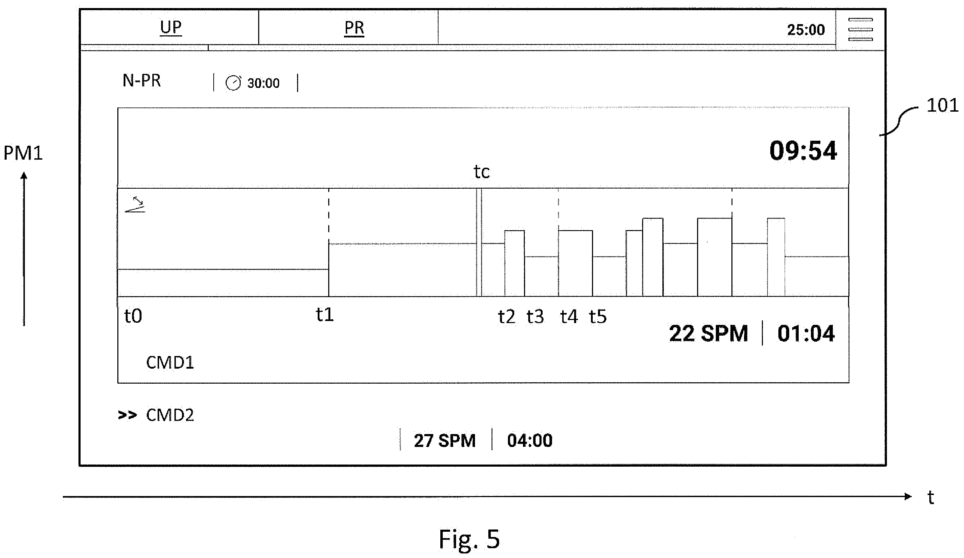

An example of such display is shown in Fig. 5.

Alternatively to the first screen UP, the control unit 101 suggests to the trainer user a second screen PR representative of a profile of a training program in progress on the plurality of exercise machines 500.

It is worth noting that the switch between the first screen UP and the second screen PR is possible by selecting the respective symbol (UP or PR) as shown at the top left in figure 4 and in figure 5.

The second screen PR comprises a chart in the middle having the time t on the abscissa and a first parameter PM1 of the exercise machine 200 which can be controlled during its operation, in the respective unit of measurement, on the ordinate, e.g. if the exercise machine is a treadmill, the gradient or inclination of the treadmill.

Along the abscissa are the initial time t0 and successive instants of time t1-t5 at which the first parameter PM1 of the exercise machine 200 which can be controlled during its operation assumes different values.

Also along the abscissa are a vertical bar, indicated by reference tc, representative of the current instant of time at which the users are during the performance of the training program on the respective exercise machine 200.

The vertical bar is located between two successive instants of time t1 and t2.

On the second screen, in addition to the name of the profile of the training program N-PR, there is a first piece of information CMD1 representative a first suggestion to provide to the user in the current instant of time tc (e.g. "keep up the pace") and a second piece of information CMD2 representative of a second suggestion to provide to the user starting from the instant of time in which the value of the first parameter PM1 will be changed (in the example in figure 5, this variation will occur in the instant of time t2).

Turning back in general to the system according to the present invention, it is worth noting that the control instructions of the operation of the exercise machine, in an embodiment, are provided by the control unit 101, both those imparted in response to a command by the trainer user (stop, pause, restart, end of training) and those imparted automatically by the control unit (synchronization, automatic end of training command), and those imparted as function of the training program profile (variation of parameters of the exercise machine which can be controlled during operation).

It is worth noting that this occurs regardless of whether the exercise machines have received the training program loaded by the trainer user by the control unit 101 or not.

According to a further embodiment, alternative to the previous one, the control instructions of the operation of the exercise machine 200 imparted by the control unit 101 as a function of the profile of the training program (variation of parameters of the exercise machine 200 which can be controlled during operation) can be automatically executed directly by the exercise machines.

This is possible if the plurality of exercise machines 500 have received the training program selected by the trainer user by the control unit 101 and are able to run it autonomously.

As mentioned above, the control unit 101 is also configured to send to the plurality of exercise machines 500, by means of the communication network data NTW, an end of training command, in the moment in which the total time of the training program end (automatic end of training command) or in the moment in which the trainer user imparts an end of training command on the control unit 101 (manual end of training command).

After sending the end of training command, at the end of the training, the control unit 101 is configured to store a summary of the training class in the respective memory module 103.

It is worth noting that "summary of the training class" means a combination of overall information, such as, for example, start of training time, end of training time and so on.

In an embodiment, in combination with the previous one, the control unit 101 is configured to send a summary of the training class to a remote database of the gym or club, e.g. by means of the Internet.

In an embodiment, either alternative to or in combination with the previous one, the control unit 101 is configured to send the summary of the training class to the remote electronic processor 300, e.g. by means of the Internet, in order to store it inside of the memory unit 302 of the remote electronic processor 300 in the trainer user's account.

Turning back in general to the system 100, we will now describe some features of an exercise machine 200 of the plurality of exercise machines 500.

The exercise machine 200 is configured to receive the start of training command from the control unit 101 by means of the communication network data NTW.

As mentioned above, it is worth noting that the exercise machine 200, until the start of training command is received, is locked and ready to be only used in the training class with which was associated.

The exercise machine 200 is configured to begin the training upon reception of the start of training command.

In an embodiment, in combination with the previous one, the exercise machine 200 is configured to receive at least one control instruction of the operation of each exercise machine 200 from the control unit 101 by means of the communication network data NTW, during operation of the plurality of exercise machines 500.

Examples of control instructions of the operation of each exercise machine were shown above.

In an embodiment, in order to receive at least one control operation instruction, the exercise machine 200 is configured to periodically send (e.g. once a second) to the control unit 101 a respective request to receive a control instruction of the operation and training data of the user on the exercise machine 200 recorded by the exercise machine during its operation.

The training data was defined above.

It is worth repeating that the control instructions of the operation of the exercise machine received from the control unit 101 may be executed automatically by the exercise machine 200, if the control instruction of the operation of the exercise machine 200 is the variation of a parameter of the exercise machine 200 which can be controlled during its operation (e.g. if the exercise machine 200 is a treadmill, the variation of the gradient and/or of the rotation speed of the treadmill).

It is worth noting that in an embodiment, in which automatic execution of the variation of a first parameter of the exercise machine 200 which can be controlled during its operation is envisaged by the exercise machine 200, the exercise machine 200 is advantageously configured to provide to the user, by means of the respective display unit 204, a piece of information representative of one or more preferred values (or one or more ranges of preferred values) of a second parameter of the exercise machine 200 which can be controlled during its operation, determined on the basis of the value of the first parameter of the exercise machine 200 which can be controlled during its operation.

For example, if the exercise machine 200 is a treadmill and the first parameter of the exercise machine 200 which can be controlled during its operation is the gradient of the treadmill, the exercise machine 200 is configured to control the gradient of the treadmill by means of the actuation device 206 and to advantageously provide to the user, by means of the respective display unit 204, a piece of information representative of one or more preferred values (or one or more ranges of preferred values) of the rotation speed of the treadmill (as the second parameter of the exercise machine 200 which can be controlled during its operation), determined on the basis of the gradient value of the treadmill.

In an embodiment, the control instructions of the operation of the exercise machine received from the control unit 101 may be executed automatically by the exercise machine as a function of a respective command of the user trainer imparted to the control unit 101.

Examples of these cases were provided previously.

The exercise machine 200 is further configured to receive from the control unit 101, by means of the communication network data NTW, a piece of information representative of the training time during the performance of the training program.

The exercise machine 200 is configured to send to the control unit 101, by means of the communication network data NTW, during the performance of the training program, data representative of the operation of the exercise machine, for example:
- the value of a first parameter of the exercise machine which can be controlled during its operation or a second parameter of the exercise machine which can be controlled during its operation;
- data representative of the user's physical condition during the training (e.g. heart rate);
- the identification data of the user (e.g. the real name or a nickname).

Furthermore, in an embodiment, in combination with any of the previous ones, the exercise machine 200 is configured to receive the training program from the control unit 101 by means of the communication network data NTW.

In an embodiment, in combination with the previous one, in which the training program is run autonomously by the exercise machine 200, the exercise machine 200 is configured to automatically modify the first parameter of the exercise machine which can be controlled during operation (e.g., the gradient or inclination of the treadmill if the exercise machine is a treadmill) as a function of the training program received from the control unit 101 in a synchronized manner with the other exercise machines 200 of the plurality of exercise machines 500.

Additionally, the exercise machine 200 is configured to modify, at any time during the performance of the training program, following a manual command received from the user by means of the control interface 203 with which the exercise machine 200 is provided, the value of the first parameter of the exercise machine 200 which can be controlled during operation (e.g. the gradient or inclination of the treadmill, if the exercise machine 200 is a treadmill) if the user is unable to keep up the value of the first parameter of the exercise machine 200 which can be controlled during operation, set automatically according to the training program.

Additionally, the exercise machine 200 is configured to modify, at any time during the performance of the training program, following a manual command received from the user by means of the control interface 203 with which the exercise machine 200 is provided, the value of the second parameter of the exercise machine 200 which can be controlled during operation (e.g., the rotation speed of the treadmill, if the exercise machine 200 is a treadmill) if the user is unable to keep up the value of the second parameter of the exercise machine 200 which can be controlled during operation, set automatically according to the training program.

In the moment in which, in the profile of the training program, a successive interval of time starts with a set value of the first parameter of the exercise machine 200 which can be controlled during operation and/or the second parameter of the exercise machine 200 which can be controlled during operation assigned with it, the exercise machine 200 is configured to align the value of the first parameter of the exercise machine 200 which can be controlled during operation and/or the value of the second parameter of the exercise machine 200 which can be controlled during operation varied manually by the user to the respective values of the profile of the training program selected by the trainer user.

Furthermore, the exercise machine 200 is configured to show to the user, by means of the display unit 204 with which the exercise machine 200 is provided, a profile of the first parameter of the exercise machine which can be controlled during operation provided for in the loaded training program with an instantaneous indication of a current point the user is at as time elapses.

Furthermore, the exercise machine 200 is configured to show to the user, by means of the display unit 204 with which the exercise machine 200 is provided, a piece of information representative of one or more preferred values (or one or more ranges of preferred values) of the second parameter of the exercise machine which can be controlled during the operation of the exercise machine (in the case of a treadmill, the second parameter of the exercise machine which can be controlled during operation is, for example, the rotation speed of the treadmill) determined on the basis of the value of the first parameter of the exercise machine which can be controlled 200 during operation of the machine.

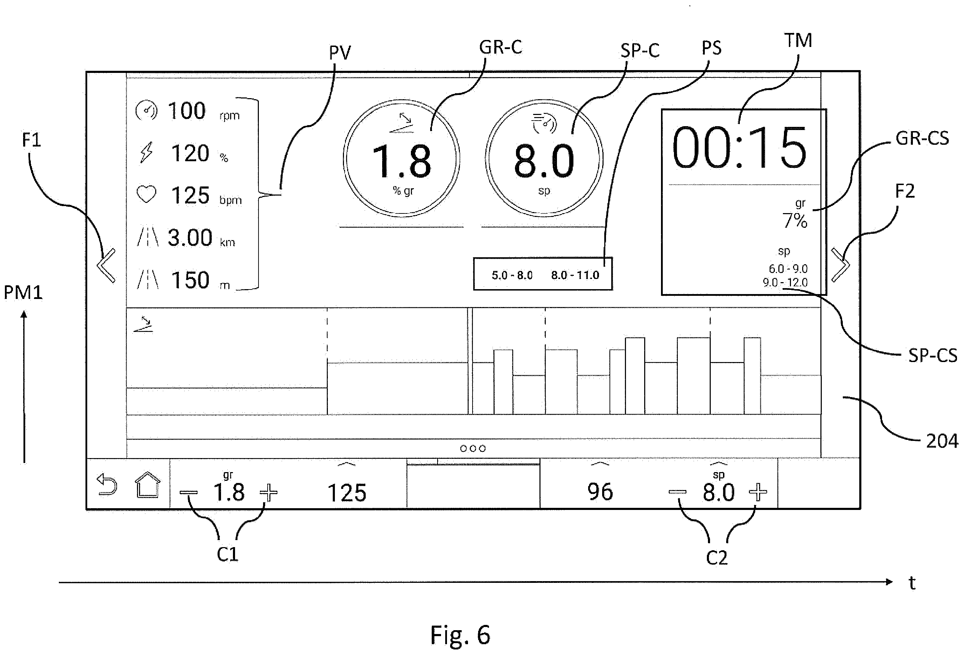

An example of such display is shown in Fig. 6.

The display unit 204 of the exercise machine 200 suggests a third screen S1 to the user representative of the training data of the user during the execution of the training program.

For example, the third screen S1 shows to the user the current value GR-C of the first parameter GR of the machine which can be controlled during the operation of the exercise machine (in the case of a treadmill, the gradient or inclination) and the current value SP-C of the second parameter SP of the exercise machine operation which can be controlled during the operation of the exercise machine (in the case of a treadmill, the rotation speed).

Additionally, the third screen S1 comprises a chart in the middle having the time t on the abscissa and a first parameter PM1 of the exercise machine which can be controlled during its operation, in the respective unit of measurement, on the ordinate, e.g. if the exercise machine is a treadmill, the gradient or inclination of the treadmill. Such graph is entirely similar to that described with reference to figure 5.

Furthermore, the third screen S1 shows icons to the user representative of first commands C1 to manually vary the first parameter GR of the exercise machine which can be controlled during the operation of the exercise machine and representative of second commands C2 to manually vary the second parameter SP of the exercise machine which can be controlled during the operation of the exercise machine.

Furthermore, the third screen S1 shows to the user a plurality of current values PV of data representative of the user's physical condition during the training.

Still, the third screen S1 comprises a first arrow F1 to go to a specific screen representative of the first parameter GR of the exercise machine which can be controlled during the operation of the exercise machine and a second arrow F2 to go to a specific screen of the second parameter SP of the exercise machine which can be controlled during the operation of the exercise machine.

Furthermore, the third screen S1 further comprises a piece of information PS representative of one or more preferred values (or one or more ranges of preferred values) of the second parameter SP of the exercise machine 200 which can be controlled during the operation of the exercise machine 200 determined on the basis of the value of the first parameter GR of exercise machine 200 which can be controlled during the operation of the exercise machine 200.

Again, the third screen S1 comprises information representative of the training program in the successive time interval, i.e.:
- the time TM left before the end of the current interval of time in which the exercise machine 200 is controlled with the current value GR-C of the first parameter GR of the machine which can be controlled during the operation of the exercise machine (in the case of a treadmill the gradient or inclination) and the current value SP-C of the second parameter SP of the exercise machine operation which can be controlled during the operation of the exercise machine (in the case of a treadmill, the rotation speed);
- the successive value GR-CS of the first parameter GR of the machine which can be controlled during the operation of the exercise machine (in the case of a treadmill, the gradient or inclination);
- the successive value SP-CS of the second parameter SP of operation of the exercise machine which can be controlled during the operation of the exercise machine (in the case of a treadmill, the rotation speed).

Turing back in general to the system in figure 1, the exercise machine 200 is also configured to receive from the control unit 101, by means of the communication network data NTW, an end of training command, in the moment in which the total time of the training program ends or in the moment in which the trainer user imparts an end of training command on the control unit 101.

In an embodiment, at the end of the training, following the reception of the end of training command, the exercise machine 200 is configured to send to the remote electronic processor 300, e.g. by means of the Internet, the results of the training performed by the user on the exercise machine 200, in order to store it inside the memory unit 302 of the remote electronic processor 300 in the account of the user (if the user has previously been authenticated on the exercise machine).

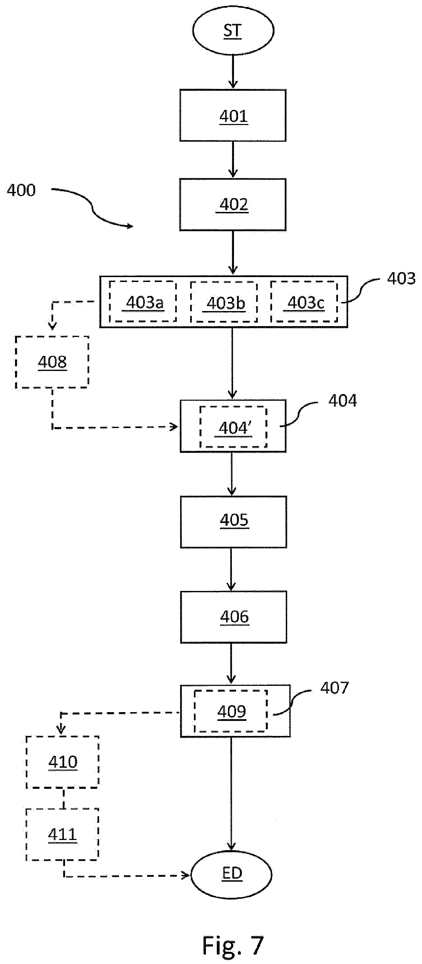

A method 400 for managing a training of users on a plurality of exercise machines, hereinafter also managing method or simply method, according to an embodiment of the present invention, will now described also with reference to figure 7.

The method 400 comprises a symbolic step of starting ST.

The system 400 comprises a step of providing 401 a control unit 101 of a training of users on a plurality of exercise machines.

The control unit 101 can be accessed by a trainer user responsible for managing the training class on a plurality of exercise machines.

The control unit 101, according to various embodiments, has been described above and is not here described for the sake of brevity of description.

The method 400 further comprises a step of providing 402 a plurality of exercise machines 500 operatively connected to the control unit 101 by means of a data communication network NTW.

As mentioned above, the plurality of exercise machines 500 are distributed in the same location so that they can be used by users for performing a training class under the guidance and/or supervision of the trainer user employing the control unit 101.

The plurality of exercise machines 500, according to various embodiments, was described above and is not here described for the sake of brevity of description.

As previously mentioned, the plurality of exercise machines 500 are locked and ready to be only used in the training class while waiting to receive a start of training command from the control unit 101, as will be described below.

The method 400 further comprises a step of loading 403, by the control unit 101, in a respective memory module 103 of the control unit 101, a training program to be performed for the users of the training class chosen by the trainer user from various training programs.

In an embodiment, shown in figure 7 with dashed lines, the step of loading 403 comprises a step of receiving 403a, by the control unit 101, training programs stored in the respective memory module 103.

In an alternative embodiment, also shown in the figure with dashed lines, the step of loading 403 comprises a step of receiving 403b, by means of the control unit 101, ome or more training programs from an electronic device of the trainer user.

In more detail, it is worth noting that the one or more training programs are created by the trainer user on the electronic device of the trainer user by means of a specific software application (APP).

In a further alternative embodiment, also shown in figure 7 by dashed lines, the step of loading 403 comprises a step of receiving 403c, by means of the control unit 101, training programs from a remote electronic processor 300 (if provided), e.g. by means of the Internet or other remote database of the gym or club, by means of the communication network data NTW.

Turning back to the embodiment in figure 7, the method 400 further comprises sending 404, by the control unit 101, by means of the data communication network NTW, a start of training command to the plurality of exercise machines 500.

In an embodiment, shown in figure 7 with dashed lines, the step of sending 404 comprises a step of showing 404' to the trainer user, by the control unit 101, e.g. on a display module 105 with which with the control unit 101 is provided, a start of training command.

Turning back to figure 7, the method 400 comprises a step of starting 405, by the plurality of exercise machines 500, the training following the reception of the start of training command.

The method 400 further comprises a step of sending 406 to the plurality of exercise machines 500, by the control unit 101, by means of the data communication network NTW, at least one control instruction of the operation of each exercise machine 200 of the plurality of exercise machines 500.

The definitions of the training program and of said at least one control instruction of the operation of the exercise machine were previously provided and here are not repeated for the sake of brevity of description.

In an embodiment, the step of sending 406 is performed by the control unit 101, in following a manual command of the trainer user.

In this embodiment, the step of sending 406 is performed by the control unit 101, to provide at least one instruction for controlling the operation of each exercise machine to the plurality of exercise machines 500, such as a stop command, a pause command, a restart command, an end of training command, a synchronization command.

In an embodiment, the step of sending 406 is performed, by the control unit 101, as a function of a profile of the training program selected by the trainer user.

In greater detail, this step of sending 406 is performed, by the control unit 101, to provide to the plurality of exercise machines 500 a control instruction of the operation of each exercise machine, such as the variation of a parameter of the exercise machine 200 which can be controlled during operation.

Turning back to the step of sending 406 the at least one control instruction of the operation of an exercise machine, it is worth noting that in an embodiment the step of sending 406 is performed, by the control unit 101, in response to the reception of a respective request to receive a control instruction of the operation of an exercise machine 200 and training data of each user on the respective exercise machine 200, recorded by the exercise machine 200 during its operation, sent periodically (for e.g. once a second) by each exercise machine 200 of the plurality of exercise machines 500.

The training data were defined above.

Turning back to figure 7, the method 400 comprises the step of controlling 407 the operation of each exercise machine 200, by each exercise machine 200 of the plurality of exercise machines 500, on the basis of the at least one control instruction of the operation of each exercise machine 200 received from the control unit 101.

It is worth noting that step of controlling 407 is performed by each exercise machine 200 of the plurality of exercise machines 500, if the control unit 101, according to the training program loaded in response to this request, sends to the exercise machine a control instruction of the operation of the exercise machine.

In a further embodiment, shown in figure 7 with a dashed line, between the step of loading 403, by the control unit 101, a training program to be performed by the training class users and the step of sending 404, by the control unit 101, a start of training command to the plurality of exercise machines 500, the method 400 comprises a step of sending 408, by the control unit 101, the training program chosen by the trainer user to the plurality of exercise machines 500, by means of the data communication network NTW.

In this embodiment, as shown in figure 7 by dashed lines, the step of controlling 407 comprises a step of performing 409 by each exercise machine 200 of the plurality of exercise machines 500, the training program received from the control unit 101.

In this embodiment, the at least one control instruction of the operation of each exercise machine 200 such as the variation of a parameter of the exercise machine which can be controlled during operation is executed independently by each exercise machine 200.

In an embodiment, if the exercise machine 200 of the plurality of exercise machines 500 is a treadmill, said exercise machine parameter which can be controlled during the operation being the gradient of the treadmill, the step of controlling 407 being performed by controlling an actuation device 206 with which each exercise machine 200 is provided configured to receive a treadmill gradient variation command from a respective data processing unit 201.

It is worth noting that in this embodiment, the step of sending 406 is performed, by the control unit 101, to provide at least one instruction for controlling the operation of each exercise machine to the plurality of exercise machines 500, such as a stop command, a pause command, a restart command, an end of training command, a synchronization command.

In an embodiment, not shown in the figures, the method 400 further comprises a step of providing, by the control unit 101, a piece of information representative of the training time during the performance of the training program to the plurality of exercise machines 500, by means of the data communication network NTW.

In an embodiment, shown in figure 7 with a dashed line, the method 400 further comprises a step of sending 410 to the plurality of exercise machines 500, by the control unit 101, via the communication network data NTW, an end of training command.

It is worth noting that the end of training command can be sent in the moment in which a total training time of the training program ends or when the trainer user imparts an end of training command on the control unit 101.

In this embodiment, as shown in figure 7 by dashed lines, the method 400 further comprises a step of ending 411, by the plurality of exercise machines 500, the training following the reception of the end of training command.

Turning back in general to the embodiment in figure 7, the method 400 ends with a symbolic step of ending ED.

In an embodiment, not shown in the figures, the method 400 further comprises a step of showing to the trainer user, by the control unit 101, by means of the respective display module 105 with which the control unit is provided 101, a list of the users participating in the training program, wherein, the user identification data and an indication representing whether a user can follow a profile of a first parameter of the exercise machine which can be controlled during operation or not, according to the loaded training program, are associated with each user.

In greater detail, the aforesaid step of showing comprises the steps of:
- receiving a value of said first parameter of the exercise machine 200 which can be controlled during its operation (e.g. the gradient of a treadmill), during the training of a user on the exercise machine 200;
- comparing such received value with the value of said first parameter of the exercise machine 200 which can be controlled during its operation envisaged by the training program in order to determine a deviation;
- if such deviation is different from zero, moving a line inside the plurality of lines PR corresponding to such user to the first place of the plurality of lines PR, possibly by adding a graphic symbol SG by the side of at least either one or a combination of: a sequential number 01, a respective real name RN1 and a respective nickname NK1 of such user.

In an embodiment, not shown in figure 7, the method 400 comprises a step of providing a remote electronic processor 300 (cloud) operatively connected to the control unit 101 and the plurality of exercise machines 500.

The remote electronic processor 300 was described above and is not repeated here for the sake of brevity of description.

In an embodiment, in combination with previous ones and not shown in figure 7, following the sending of the end of training command, at the end of the training, the method 400 comprises a step of storing, by the control unit 101, in the respective memory module 103, a summary of the training class.

It is worth noting that "summary of the training class" means a combination of overall information, such as, for example, start of training time, end of training time and so forth.

In an embodiment, in combination with the previous one, the method 400 comprises a step of sending, by the control unit 101, to a remote database of gym or club, e.g. by means of the Internet, the summary of the training class.

In an embodiment, either alternatively to or in combination with the previous one, the method 400 comprises a step of sending, by the control unit 101, to the remote electronic processor 300, e.g. by means of the Internet, the summary of the training class in order to store it inside of the memory unit 302 of the remote electronic processor 300 in the account of the trainer user.

In an embodiment, in combination with the previous ones, at the end of the training, following the reception of the end of training command, the method 400 comprises a step of sending, by each exercise machine 200 of the plurality of exercise machines 500, to the remote electronic processor 300, e.g. by means of the Internet, the results of the training performed by the user on the exercise machine 200, in order to store it inside the memory unit 302 of the remote electronic processor 300 in the account of the user (if the user has previously been authenticated on the exercise machine).

According to another embodiment, in combination with any one of the embodiments described above, the method 400 comprises a step of receiving, by each exercise machine 200 of the plurality of exercise machines 500, from the control unit 101 by means of the communication network data NTW, the training program selected by the trainer user.

Furthermore, in an embodiment, the method 400 comprises a step of receiving, by each exercise machine 200 of the plurality of exercise machines 500, from the control unit 101, by means of the communication network data NTW, a start of training command.

Furthermore, in an embodiment, the method 400 comprises a step of receiving, by each exercise machine 200 of the plurality of exercise machines 500, from the control unit 101, by means of the communication network data NTW, a piece of information representative of the training time during the performance of the training program.

In an embodiment, in combination with any of the previous ones, the method 400 comprises a step of sending, by each exercise machine 200 of the plurality of exercise machines 500, to the control unit 101, by means of the data communication network NTW, during the performance of the training program, a request of control instructions of the operation of the exercise machine and training data of the user on the exercise machine, such as at least either one or a combination of:
- the value of a first parameter of the exercise machine which can be controlled during operation;
- the value of a second parameter of the exercise machine which can be controlled during operation;
- data representative of the user's physical condition during the training;
- identification data of the user (e.g. the name or a nickname).

Additionally, in an embodiment, in combination with any of the previous ones, the method 400 comprises a step of manually modifying, at any moment during the performance of the training program, following a command received from the user via a control interface 203 with which an exercise machine 200 of the plurality of exercise machines 500 is provided, the value of the first parameter of the exercise machine which can be controlled during operation (e.g. the gradient of inclination of the treadmill if the exercise machine is a treadmill) or of a second parameter of the exercise machine which can be controlled during operation (e.g. the rotation speed of the treadmill if the exercise machine is a treadmill) if the user cannot keep the value of the first parameter of the exercise machine which can be controlled during operation or the value of the second parameter of the exercise machine which can be controlled during operation, set automatically during the training program chosen by the trainer user.

Furthermore, according to an embodiment of the invention, in combination with any one of the embodiments described above, the method 400 includes a step of showing to the user, by the respective exercise machine 200, by means of the display unit 204 with which the exercise machine 200 is provided, a profile of the first parameter of the exercise machine which can be controlled during its operation and/or the profile of the second parameter of the exercise machine which can be controlled during its operation, provided in the training program loaded with an instantaneous indication of a current point the user is at as time elapses.

Furthermore, according to an embodiment, in combination with any one of the previous ones, the method 400 comprises a step of providing the user, by the respective exercise machine 200 by means of a display unit 204 with which the exercise machine 200 is provided, with a piece of information representative of one or more preferred values (or one or more intervals of preferred values) of the second parameter of the exercise machine 200 which can be controlled during its operation, determined on the basis of the value of the first parameter of the exercise machine 200 which can be controlled during its operation.

In an embodiment, in combination with any one of the preceding ones, the method 400 comprises a step of receiving, by each exercise machine 200 of the plurality of exercise machines 500, from the control unit 101, by means of the communication network data NTW, an end of training command, in the moment in which the total training time program ends or when the trainer user imparts an end of training command on the control unit 101.

An example of operation of the system 100 in an embodiment thereof, by implementing the method 400, according to an embodiment thereof will now be described with reference to the aforementioned figures.

According to the present example, the plurality of exercise machines 500 comprises treadmills.

The user trainer loads on the control unit 101 a training program chosen from the training programs previously stored in the memory module 103 of the control unit 101.

The trainer user sends, by means of the control unit 101 and the data communication network NTW, the loaded training program to the plurality of exercise machines 500 belonging to the training class, previously associated with the control unit 101 by storing the IP address of the control unit 101 on each exercise machine 200 of the plurality of exercise machines 500.

Subsequently, the control unit 101 shows the start of training command to the trainer user, by means of the display module 104.

The trainer user actuates the start of training command on the control unit 101.

The control unit 101 sends the start of training command to the plurality of exercise machines 500, by means of the communication network data NTW.

The plurality of exercise machines 500 (treadmills) starts the training.

The training of the users in the training class begins on all treadmills which, until the start of training command was received, were locked and ready to run the training class.

Each exercise machine 200 periodically sends (e.g. once a second) to the control unit 101 by means of the communication network data NTW, a request for control instructions of the operation of the treadmill and training data of the respective user on the exercise machine 200, recorded by the exercise machine 200 during its operation.

If it is envisaged in the training program selected by the trainer user, or as a result of the manual control of the trainer user or as a function of the profile of the training program, the control unit 101 sends at least one control instruction of the operation of each exercise machine to the plurality of exercise machines 500.

Each exercise machine 200 of the plurality of exercise machines 500 controls the respective operation on the basis of the at least one control instruction of the operation of each exercise machine 200 received from the control unit 101.

In particular, each exercise machine 200 of the plurality of exercise machines 500 can vary, on the basis of at least one received control instruction of the operation, the first parameter of the exercise machine which can be controlled during operation, for example the gradient or gradient of the treadmill.

During the execution of the training program, the control unit 101 shows to the trainer user, by means of a respective display module 105 with which the control unit 101 is provided, a list of the users participating in the training program, in which, the user identification data and an indication representing whether a user can follow a profile of a first parameter of the exercise machine which can be controlled during operation or not, according to the loaded training program, are associated with each user.

During the execution of the training program, a user may manually modify, at any time, by means of the control interface 203 with which the exercise machine 200 is provided, the value of the first parameter of the exercise machine which can be controlled during operation (e.g. the gradient or inclination of the treadmill if the exercise machine is a treadmill), if the user is unable to keep up the value of the first parameter of the exercise machine 200 which can be controlled during operation, set automatically according to the training program.

In the moment in which, in the profile of the training program, a subsequent time interval starts with assigned a set value of the first parameter of the exercise machine which can be controlled during operation, the first parameter of the exercise machine which can be controlled during operation varied manually by the user will be aligned with the respective value of the profile of the training program selected by the trainer user.

According to a specific example, while performing the training, the users of the training class can run on treadmills for a first period of time, e.g. 5 minutes at a rotation speed of the treadmill of 8-10 km/h (which can be managed manually by the user on the exercise machine) with a gradient of the treadmill of 2% (managed automatically by the exercise machine as a function of the profile of the training program selected by the trainer user).

The trainer user sends a pause command, by means of the control unit 101, to the plurality of exercise machines 500.

Following the reception of the pause command sent by the control unit 101, each exercise machine 200 controls the respective electric motor to decelerate the treadmill to take it to a zero-rotation speed following a set deceleration ramp. The gradient of each treadmill instead remains that of the profile of the training program chosen by the trainer user assigned in the instant in time in which the pause command is imparted.

Users step off their respective treadmill and stand next to it to perform floor exercises as instructed by the trainer user, e.g. push-ups for a prescribed time interval (e.g., 30 seconds).

The users then step back onto their respective treadmill.

The trainer user sends a restart command, by means of the control unit 101, to the plurality of exercise machines 500.

Following the reception of the restart command sent by the control unit 101, each exercise machine 200 controls the respective electric motor to accelerate the treadmill to take it to a set rotation speed (e.g. the one at which was before the previous deceleration ramp) following a set acceleration ramp. The gradient of each treadmill instead remains that of the profile of the training program chosen by the trainer user assigned in the instant in time in which the pause command is imparted.

Once the training is finished, the trainer user imparts an end of training command on the control unit 101.

The control unit 101 sends, by means of the data communication network NTW, an end of training command to the plurality of exercise machines 500.

The training program of the users in the training class ends.

At the end of the training, the control unit 101 stores a summary of the training class in a respective memory module 103.

The control unit 101 sends a summary of the training class to a remote database of the gym or club, e.g. by means of the Internet.

The control unit 101 also sends the summary of the training class to the remote electronic processor 300 (where provided), e.g. by means of the Internet, in order to store it inside the memory unit 302 of the remote electronic processor 300 in the trainer user's account.

Each exercise machine 200 of the plurality of exercise machines 500 sends the user's training results on the exercise machine 200 to the remote electronic processor 300, if provided, in order to store the training results in the memory unit 302 of the remote electronic processor 300 in the user's account (if the latter was previously authenticated on the exercise machine).

It is apparent that the object of the invention is fully achieved because the method and system according to the present invention allows the control of a plurality of exercise machines by the control unit managed by the trainer user.

Indeed, the automatic loading, on both the control unit and on the plurality of exercise machines, of the same training program advantageously allows the synchronized performance of the training program in which, for example, instructions for controlling the operation of an exercise machine, such as stop, pause, restart, end of training commands, or variation of a first parameter of the exercise machine which can be controlled during the operation of the exercise machine (gradient of a treadmill) or a second parameter of the exercise machine which can be controlled during operation (rotation speed of a treadmill) are performed in automatic and synchronized manner across all exercise machines employed by users of the class.

In this manner, the risk that a command, vocal or other, of the trainer user to vary, for example, the gradient of a treadmill is either not heard, not performed successfully or, if heard, is performed with considerable delay, is greatly reduced.

Therefore, the method and system for managing a training of users on exercise machines according to the present invention allows to avoid as much as possible problems to the users during the user's training, such as accidents or excessive fatigue.

Furthermore, the method and system for managing a training for users on a plurality of exercise machines according to the present invention advantageously allows to reduce as much as possible the manual adjustments of the respective exercise machine by the user during the training class, whereby increasing the level of accuracy, synchronization and coordination of the entire training class.

Still, it is advantageously possible for the trainer user to perform a training program which provides moments of use of the exercise machine and moments of exercises on the floor without using the exercise machine in manner as coordinated as possible.

Furthermore, if a user is still breathless because of excessive effort, the user can resort to controlling the exercise machine in order to be able to recover physical energy and catch his or her breath and subsequently, when the exercise machine is aligned back to the loaded training program, successfully continue the training program followed by the class.

A person skilled in art may make changes and adaptations to the method and respective system described above or can replace elements with others which are functionally equivalent to satisfy contingent needs without departing from the scope of protection of the appended claims. All the features described above as belonging to one possible embodiment may be implemented independently from the other described embodiments.

## Claims

1. A method (400) for managing a training of users on a plurality of exercise machines (500), comprising steps of:
- providing (401) a control unit (101) of a user training class on a plurality of exercise machines (500), the control unit (101) being accessible by a trainer user in charge of managing a training class on a plurality of exercise machines (500);
- providing (402) a plurality of exercise machines (500) operatively connected to the control unit (101) by means of a data communication network (NTW);
- loading (403), by the control unit (101), in a respective memory module (103) of the control unit (101), a training program to be performed for the users of the training class chosen by the trainer user from various training programs;
- sending (404), by the control unit (101), by means of the data communication network (NTW), a start of training command to the plurality of exercise machines (500);
- unlocking, by the control unit (101) using the start of training command, the plurality of exercise machines (500) which, until the start of training command was received, are locked and ready to be used only in the training class with which they were associated;
- starting (405), by the plurality of exercise machines (500), the training following the reception of the start of training command;
- sending (406) to the plurality of exercise machines (500), by the control unit (101), by means of the data communication network (NTW), at least one control instruction of the operation of each exercise machine (200) of the plurality of exercise machines (500);
- controlling (407) the operation of each exercise machine (200), by each exercise machine (200) of the plurality of exercise machines (500), on the basis of the at least one control instruction of the operation of each exercise machine (200) received by the control unit (101).

2. A method (400) according to claim 1, wherein the step of sending (406) is performed, by the control unit (101), following a manual command of the trainer user, the step of sending (406) being performed, by the control unit (101), to provide the plurality of exercise machines (500) with the at least one control instruction of the operation of each exercise machine, such a control belonging to the group comprising: a stop command, a pause command, a restart command, an end of training command, a synchronization command.

3. A method (400) according to claim 1, wherein the step of sending (406) is performed, by the control unit (101), as a function of a profile of the training program chosen by the trainer user, the step of sending (406) being performed, by the control unit, (101), to provide the plurality of exercise machines (500) with the at least one control instruction of the operation of each exercise machine (200), such as the variation of a parameter of the exercise machine (200) which can be controlled during operation.

4. A method (400) according to any one of the preceding claims, further comprising, between the step of loading (403), by the control unit (101), a training program to be performed for the training class users and the step of sending (404), by the control unit (101), a start of training command to the plurality of exercise machines (500), a step of sending (408), by the control unit (101), the training program chosen by the trainer user to the plurality of exercise machines (500), by means of the data communication network (NTW).

5. A method (400) according to claim 4, further comprising a step of performing (409), by each exercise machine (200) of the plurality of exercise machines (500), the training program received from the control unit (101), the at least one control instruction of the operation of each exercise machine (200), such as the variation of a parameter of the exercise machine which can be controlled during operation, being performed autonomously by each exercise machine (200).

6. A method (400) according to claim 5, wherein each exercise machine (200) of the plurality of exercise machines (500) is a treadmill, said exercise machine parameter which can be controlled during operation being the gradient of the treadmill, the step of controlling (407) being performed by controlling an actuation device (206) with which each exercise machine (200) is provided configured to receive a treadmill gradient variation command from a respective data processing unit (201) of the exercise machine (200).

7. A method (400) according to any one of the preceding claims, wherein the step of sending (406) the at least one control instruction of the operation of an exercise machine (200) is performed, by the control unit (101), in reply to the reception of a respective request to receive a control instruction of the operation of an exercise machine (200) and of training data of each user on a respective exercise machine (200), recorded by the exercise machine (200) during its operation, periodically sent by each exercise machine (200) of the plurality of exercise machines (500).

8. A method (400) according to any one of the preceding claims, further comprises a step of providing, by the control unit (101), a piece of information representative of the training time during the performance of the training program to the plurality of exercise machines (500), by means of the data communication network (NTW).

9. A method (400) according to any one of the preceding claims, further comprising a step of showing to the trainer user, by the control unit (101), by means of a respective display module (105) with which the control unit is provided (101), a list of the users taking part in the training program, wherein, the user identification data and an indication representing the fact that a user can follow a profile of a first parameter of the exercise machine which can be controlled during operation or not, according to the loaded training program, are associated with each user, said step of showing comprising steps of:
- receiving a value of said first parameter of the exercise machine (200) which can be controlled during its operation, during a user's training on the exercise machine (200);
- comparing such received value with the value of said first parameter of the exercise machine (200) which can be controlled during its operation envisaged by the training program in order to determine a deviation;
- if such deviation is different from zero, moving a line inside the plurality of lines (PR) corresponding to such user to the first place of the plurality of lines (PR), adding a graphic symbol (SG) by the side of at least either one or a combination of: a sequential number (01), a respective real name (RN1) and a respective nickname (NK1) of such user.

10. A method (400) according to any one of the preceding claims, further comprising a step of sending, by each exercise machine (200) of the plurality of exercise machines (500), to the control unit (101), by means of the data communication network (NTW), during the performance of the training program, a request of control instructions of the operation of the exercise machine (200) and training data of the user on the exercise machine (200), such as at least either one or a combination of:
- the value of a first parameter of the exercise machine which can be controlled during operation;
- the value of a second parameter of the exercise machine which can be controlled during operation;
- data representative of the user's physical condition during the training;
- user's identification data.

11. A method (400) according to any one of the preceding claims, further comprising a step of manually modifying, at any moment during the performance of the training program, following a command received from the user via a control interface (203) with which an exercise machine (200) of the plurality of exercise machines (500) is provided, a value of a first parameter of the exercise machine which can be controlled during operation or of a second parameter of the exercise machine which can be controlled during operation if the user cannot keep the value of the first parameter of the exercise machine which can be controlled during operation or the value of the second parameter of the exercise machine which can be controlled during operation, set automatically during the training program chosen by the trainer user.

12. A method (400) according to any one of the preceding claims, comprising a step of providing the user, by the respective exercise machine (200) by means of a display unit (204) with which the exercise machine (200) is provided, with a piece of information representative of one or more preferred values of a second parameter of the exercise machine (200) which can be controlled during its operation, determined on the basis of the value of the first parameter of the exercise machine (200) which can be controlled during its operation.

13. A method (400) according to any one of the preceding claims, further comprising steps of:
- sending (410), by the control unit (101), an end of training command to the plurality of exercise machines (500), by means of the data communication network (NTW);
- ending (411), by the plurality of exercise machines (500), the training following the reception of the end of training command.

14. A method (400) according to any one of the preceding claims, comprising a step of providing a remote electronic processor (300) operatively connected to the control unit (101) and to the plurality of exercise machines (200), the method (400), after sending an end of training command, at the end of the training, comprising steps of:
- storing, by the control unit (101), a summary of the training class in a respective memory module (103);
- sending, by the control unit (101), to the remote electronic processor (300), the summary of the training class in order to store it in a control unit (302) of the remote electronic processor (300) in a personal trainer user's account.

15. A method (400) according to claim 14, comprising, following the reception of the end of the training command, a step of sending, by each exercise machine (200) of the plurality of exercise machines (500), to the remote electronic processor (300), training results performed by the user on the exercise machine (200), in order to store them in the memory unit (302) of the remote electronic processor (300) in a user's account.

16. A system (100) for managing a training of users on a plurality of exercise machines (200), comprising:
- a control unit (101) of a training class of users on a plurality of exercise machines (500), the control unit (101) being accessible by a trainer user in charge of managing a training class on a plurality of exercise machines (500);
- a plurality of exercise machines (500) operatively connected with the control unit (101) by means of a data communication network (NTW);
the system (100) being configured to perform the method (400) for managing a training of users on a plurality of exercise machines (500) according to any one of the preceding claims from 1 to 13.

17. A system (100) according to claim 16, further comprising a remote electronic processor (300) operatively connected to the control unit (101) and to the plurality of exercise machines (500), the system (100) being configured to perform the method (400) for managing a training of users on a plurality of exercise machines (500) according to any one of the preceding claims 14 or 15.

## Patentansprüche

1. Verfahren (400) zum Verwalten eines Trainings von Benutzern an einer Mehrzahl von Trainingsmaschinen (500), umfassend die Schritte:
- Bereitstellen (401) einer Steuereinheit (101) einer Benutzer-Trainingsklasse an einer Mehrzahl von Trainingsmaschinen (500), wobei die Steuereinheit (101) von einem Trainerbenutzer zugreifbar ist, welcher verantwortlich für ein Verwalten einer Trainingsklasse an einer Mehrzahl von Trainingsmaschinen (500) ist;
- Bereitstellen (402) einer Mehrzahl von Trainingsmaschinen (500), welche betriebsmäßig mit der Steuereinheit (101) mittels eines Daten-Kommunikationsnetzwerks (NTW) verbunden sind;
- Laden (403) in ein entsprechendes Speichermodul (103) der Steuereinheit (101) durch die Steuereinheit (101) eines Trainingsprogramms, welches für die Benutzer der Trainingsklasse auszuführen ist, welches von dem Trainerbenutzer aus verschiedenen Trainingsprogrammen ausgewählt wird;
- Senden (404) einer Training-Startanweisung zu der Mehrzahl von Trainingsmaschinen (500) durch die Steuereinheit (101) mittels des Daten-Kommunikationsnetzwerks (NTW);
- Freigeben der Mehrzahl von Trainingsmaschinen (500) durch die Steuereinheit (101) unter Verwendung der Training-Startanweisung, welche, bis die Training-Startanweisung empfangen wurde, gesperrt sind und lediglich für eine Verwendung in der Trainingsklasse bereit sind, welcher sie zugeordnet wurden;
- Starten (405) des Trainings durch die Mehrzahl von Trainingsmaschinen (500) auf den Empfang der Training-Startanweisung folgend;
- Senden (406) von wenigstens einer Steueranweisung des Betriebs von jeder Trainingsmaschine (200) aus der Mehrzahl von Trainingsmaschinen (500) zu der Mehrzahl von Trainingsmaschinen (500) durch die Steuereinheit (101) mittels des Daten-Kommunikationsnetzwerks (NTW);
- Steuern (407) des Betriebs von jeder Trainingsmaschine (200) durch jede Trainingsmaschine (200) aus der Mehrzahl von Trainingsmaschinen (500) auf der Grundlage von wenigstens einer Steueranweisung des Betriebs von jeder Trainingsmaschine (200), welche von der Steuereinheit (101) empfangen wird.

2. Verfahren (400) nach Anspruch 1, wobei der Schritt des Sendens (406) durch die Steuereinheit (101) auf eine manuelle Anweisung des Trainerbenutzers folgend durchgeführt wird, wobei der Schritt des Sendens (406) durch die Steuereinheit (101) durchgeführt wird, um der Mehrzahl von Trainingsmaschinen (500) die wenigstens eine Steueranweisung des Betriebs von jeder Trainingsmaschine bereitzustellen, wobei eine derartige Steuerung zu der Gruppe gehört, welche umfasst: eine Stopp-Anweisung, eine Pause-Anweisung, eine Neustart-Anweisung, eine Training-Endanweisung, eine Synchronisations-Anweisung.

3. Verfahren (400) nach Anspruch 1, wobei der Schritt des Sendens (406) durch die Steuereinheit (101) als eine Funktion eines Profils des Trainingsprogramms durchgeführt wird, welches von dem Trainerbenutzer ausgewählt wird, wobei der Schritt des Sendens (406) von der Steuereinheit (101) durchgeführt wird, um der Mehrzahl von Trainingsmaschinen (500) wenigstens eine Steueranweisung des Betriebs von jeder Trainingsmaschine (200) bereitzustellen, wie beispielsweise die Variation eines Parameters der Trainingsmaschine (200), welcher während des Betriebs gesteuert werden kann.

4. Verfahren (400) nach einem der vorhergehenden Ansprüche, ferner umfassend zwischen dem Schritt des Ladens (403) eines für die Trainingsklasse-Benutzer durchzuführenden Trainingsprogramms durch die Steuereinheit (101) und dem Schritt des Sendens (404) einer Training-Startanweisung zu der Mehrzahl von Trainingsmaschinen (500) durch die Steuereinheit (101) einen Schritt eines Sendens (408) des von dem Trainerbenutzer ausgewählten Trainingsprogramms zu der Mehrzahl von Trainingsmaschinen (500) durch die Steuereinheit (101) mittels des Daten-Kommunikationsnetzwerks (NTW).

5. Verfahren (400) nach Anspruch 4, ferner umfassend einen Schritt eines Durchführens (409) von jeder Trainingsmaschine (200) aus der Mehrzahl von Trainingsmaschinen (500) des von der Steuereinheit (101) empfangenen Trainingsprogramms, wobei die wenigstens eine Steueranweisung des Betriebs von jeder Trainingsmaschine (200), wie beispielsweise die Variation eines Parameters der Trainingsmaschine, welcher während des Betriebs gesteuert werden kann, autonom von jeder Trainingsmaschine (200) durchgeführt wird.

6. Verfahren (400) nach Anspruch 5, wobei jede Trainingsmaschine (200) aus der Mehrzahl von Trainingsmaschinen (500) ein Laufband ist, wobei der Trainingsmaschinen-Parameter, welcher während des Betriebs gesteuert werden kann, der Gradient des Laufbands ist, wobei der Schritt des Steuerns (407) durch Steuern einer Betätigungsvorrichtung (206) durchgeführt wird, mit welcher jede Trainingsmaschine (200) versehen ist, welche dazu eingerichtet ist, eine Laufbandgradient-Variationsanweisung von einer entsprechenden Datenverarbeitungseinheit (201) der Trainingsmaschine (200) zu empfangen.

7. Verfahren (400) nach einem der vorhergehenden Ansprüche, wobei der Schritt des Sendens (406) der wenigstens einen Steueranweisung des Betriebs einer Trainingsmaschine (200) von der Steuereinheit (101) in Reaktion auf den Empfang einer entsprechenden Anfrage durchgeführt wird, eine Steueranweisung des Betriebs einer Trainingsmaschine (200) und von Trainingsdaten von jedem Benutzer an einer entsprechenden Trainingsmaschine (200) zu empfangen, welche von der Trainingsmaschine (200) während ihres Betriebs aufgenommen werden, welche periodisch von jeder Trainingsmaschine (200) aus der Mehrzahl von Trainingsmaschinen (500) gesendet wird.

8. Verfahren (400) nach einem der vorhergehenden Ansprüche, ferner umfassend einen Schritt eines Bereitstellens eines Stücks von Information durch die Steuereinheit (101), welches die Trainingszeit während des Ausführens des Trainingsprogramms repräsentiert, zu der Mehrzahl von Trainingsmaschinen (500) mittels des Daten-Kommunikationsnetzwerks (NTW).

9. Verfahren (400) nach einem der vorhergehenden Ansprüche, ferner umfassend einen Schritt eines Anzeigens für den Trainerbenutzer durch die Steuereinheit (101) mittels eines entsprechenden Anzeigemoduls (105), mit welchem die Steuereinheit versehen ist (101), einer Liste der Benutzer, welche an dem Trainingsprogramm teilnehmen, wobei die Benutzer-Identifikationsdaten und ein Hinweis, welcher die Tatsache repräsentiert, dass ein Benutzer einem Profil eines ersten Parameters der Trainingsmaschine folgen kann, welcher während des Betriebs gesteuert werden kann, oder nicht, gemäß dem geladenen Trainingsprogramm jedem Benutzer zugeordnet werden, wobei der Schritt des Anzeigens die Schritte umfasst:
- Empfangen eines Werts des ersten Parameters der Trainingsmaschine (200), welcher während des Betriebs gesteuert werden kann, während eines Trainings eines Benutzers an der Trainingsmaschine (200);
- Vergleichen eines solchen empfangenen Werts mit dem Wert des ersten Parameters der Trainingsmaschine (200), welcher während ihres Betriebs gesteuert werden kann, welcher von dem Trainingsprogramm vorgesehen ist, um eine Abweichung zu bestimmen;
- wenn eine solche Abweichung von Null verschieden ist, Bewegen einer Linie innerhalb der Mehrzahl von Linien (PR), welche einem solchen Benutzer entspricht, zu einem ersten Platz aus der Mehrzahl von Linien (PR), Hinzufügen eines grafischen Symbols (SG) an der Seite von wenigstens entweder einem oder einer Kombination aus: einer sequentiellen Nummer (01), einem entsprechenden Realnamen (RN1) und einem entsprechenden Spitznamen (NK1) eines solchen Benutzers.

10. Verfahren (400) nach einem der vorhergehenden Ansprüche, ferner umfassend einen Schritt eines Sendens von jeder Trainingsmaschine (200) aus der Mehrzahl von Trainingsmaschinen (500) zu der Steuereinheit (101) mittels des Daten-Kommunikationsnetzwerks (NTW) während des Durchführens des Trainingsprogramms einer Anfrage von Steueranweisungen des Betriebs der Trainingsmaschine (200) und von Trainingsdaten des Benutzers an der Trainingsmaschine (200), wie beispielsweise wenigstens eines oder eine Kombination aus:
- dem Wert eines ersten Parameters der Trainingsmaschine, welcher während des Betriebs gesteuert werden kann;
- dem Wert eines zweiten Parameters der Trainingsmaschine, welcher während des Betriebs gesteuert werden kann;
- Daten, welche den physischen Zustand des Benutzers während des Trainings repräsentieren;
- Identifikationsdaten eines Benutzers.

11. Verfahren (400) nach einem der vorhergehenden Ansprüche, ferner umfassend einen Schritt eines manuellen Modifizierens zu jedem Zeitpunkt während des Durchführens des Trainingsprogramms, auf eine Anweisung folgend, welche von dem Benutzer mittels einer Steuerschnittstelle (203) empfangen wird, mit welcher eine Trainingsmaschine (200) aus der Mehrzahl von Trainingsmaschinen (500) versehen ist, eines Werts eines ersten Parameters der Trainingsmaschine, welcher während des Betriebs gesteuert werden kann, wenn der Benutzer den Wert des ersten Parameters der Trainingsmaschine nicht halten kann, welcher während des Betriebs gesteuert werden kann, oder des Werts des zweiten Parameters der Trainingsmaschine, welcher während des Betriebs gesteuert werden kann, welcher automatisch während des von dem Trainerbenutzer ausgewählten Trainingsprogramms festgelegt wird.

12. Verfahren (400) nach einem der vorhergehenden Ansprüche, umfassend einen Schritt eines Bereitstellens an den Benutzer durch die entsprechende Trainingsmaschine (200) mittels einer Anzeigeeinheit (204), mit welcher die Trainingsmaschine (200) versehen ist, eines Stücks von Information, welches einen oder mehrere bevorzugte Werte eines zweiten Parameters der Trainingsmaschine (200) repräsentiert, welcher während ihres Betriebs gesteuert werden kann, bestimmt auf der Basis des Werts des ersten Parameters der Trainingsmaschine (200), welcher während ihres Betriebs gesteuert werden kann.

13. Verfahren (400) nach einem der vorhergehenden Ansprüche, ferner umfassend die Schritte:
- Senden (410) durch die Steuereinheit (101) einer Training-Endanweisung zu der Mehrzahl von Trainingsmaschinen (500) mittels des Daten-Kommunikationsnetzwerks (NTW);
- Beenden (411) des Trainings durch die Mehrzahl von Trainingsmaschinen (500) auf den Empfang der Training-Endanweisung folgend.

14. Verfahren (400) nach einem der vorhergehenden Ansprüche, umfassend einen Schritt eines Bereitstellens eines entfernten elektronischen Prozessors (300), welcher betriebsmäßig mit der Steuereinheit (101) und der Mehrzahl von Trainingsmaschinen (200) verbunden ist, wobei das Verfahren (400) nach dem Senden einer Trainings-Endanweisung an dem Ende des Trainings die Schritte umfasst:
- Speichern durch die Steuereinheit (101) einer Zusammenfassung der Trainingsklasse in einem entsprechenden Speichermodul (103);
- Senden durch die Steuereinheit (101) zu dem entfernten elektronischen Prozessor (300) der Zusammenfassung der Trainingsklasse, um sie in einer Steuereinheit (302) des entfernten elektronischen Prozessors (300) in einem Account eines persönlichen Trainerbenutzers zu speichern.

15. Verfahren (400) nach Anspruch 14, umfassend auf den Empfang der Training-Endanweisung folgend einen Schritt eines Sendens durch jede Trainingsmaschine (200) aus der Mehrzahl von Trainingsmaschinen (500) zu dem entfernten elektronischen Prozessor (300) von Trainingsresultaten, welche von dem Benutzer an der Trainingsmaschine (200) durchgeführt werden, um sie in der Speichereinheit (302) des entfernten elektronischen Prozessors (300) in einem Account eines Benutzers zu speichern.

16. System (100) zum Verwalten eines Trainings von Benutzern an einer Mehrzahl von Trainingsmaschinen (200), umfassend:
- eine Steuereinheit (101) einer Trainingsklasse von Benutzern an einer Mehrzahl von Trainingsmaschinen (500), wobei die Steuereinheit (101) von einem Trainerbenutzer zugänglich ist, welcher verantwortlich für ein Verwalten einer Trainingsklasse an einer Mehrzahl von Trainingsmaschinen (500) ist;
- eine Mehrzahl von Trainingsmaschinen (500), welche betriebsmäßig mit der Steuereinheit (101) mittels eines Daten-Kommunikationsnetzwerks (NTW) verbunden sind;
wobei das System (100) dazu eingerichtet ist, das Verfahren (400) zum Verwalten eines Trainings von Benutzern an einer Mehrzahl von Trainingsmaschinen (500) gemäß einem der vorhergehenden Ansprüche von 1 bis 13 durchzuführen.

17. System (100) nach Anspruch 16, ferner umfassend einen entfernten elektronischen Prozessor (300), welcher mit der Steuereinheit (101) und der Mehrzahl von Trainingsmaschinen (500) betriebsmäßig verbunden ist, wobei das System (100) dazu eingerichtet ist, das Verfahren (400) zum Verwalten eines Trainings von Benutzern an einer Mehrzahl von Trainingsmaschinen (500) gemäß einem der vorhergehenden Ansprüche 14 oder 15 durchzuführen.

## Revendications

1. Procédé (400) pour gérer un entraînement d'utilisateurs sur une pluralité d'appareils d'exercice (500), comprenant les étapes consistant à :
- fournir (401) une unité de commande (101) d'une classe d'entraînement d'utilisateurs sur une pluralité d'appareils d'exercice (500), l'unité de commande (101) étant accessible par un utilisateur entraîneur chargé de gérer une classe d'entraînement sur une pluralité d'appareils d'exercice (500) ;
- fournir (402) une pluralité d'appareils d'exercice (500) fonctionnellement connectés à l'unité de commande (101) au moyen d'un réseau de communication de données (NTW) ;
- charger (403), par l'unité de commande (101), dans un module de mémoire respectif (103) de l'unité de commande (101), un programme d'entraînement à exécuter pour les utilisateurs de la classe d'entraînement, choisi par l'utilisateur entraîneur parmi divers programmes d'entraînement ;
- transmettre (404), par l'unité de commande (101), au moyen du réseau de communication de données (NTW), une commande de début d'entraînement à la pluralité d'appareils d'exercice (500) ;
- déverrouiller, par l'unité de commande (101) au moyen de la commande de début d'entraînement, la pluralité d'appareils d'exercice (500) qui, jusqu'à la réception de la commande de début d'entraînement, sont verrouillés et prêts à être utilisés uniquement dans la classe d'entraînement à laquelle ils étaient associés ;
- initier (405), par la pluralité d'appareils d'exercice (500), l'entraînement suite à la réception de la commande de début d'entraînement ;
- transmettre (406) à la pluralité d'appareils d'exercice (500), par l'unité de commande (101), au moyen du réseau de communication de données (NTW), au moins une instruction de commande du fonctionnement de chaque appareil d'exercice (200) de la pluralité d'appareils d'exercice (500) ;
- commander (407) le fonctionnement de chaque appareil d'exercice (200), par chaque appareil d'exercice (200) de la pluralité d'appareils d'exercice (500), sur la base de l'instruction de commande du fonctionnement de chaque appareil d'exercice (200), au moins au nombre de une, reçue par l'unité de commande (101).

2. Procédé (400) selon la revendication 1, dans lequel l'étape de transmission (406) est exécutée, par l'unité de commande (101), suite à une commande manuelle de l'utilisateur entraîneur, l'étape de transmission (406) étant exécutée, par l'unité de commande (101), pour doter la pluralité d'appareils d'exercice (500) d'au moins une instruction de commande du fonctionnement de chaque appareil d'exercice, une telle commande appartenant au groupe comprenant : une commande d'arrêt, une commande de pause, une commande de redémarrage, une commande de fin d'entraînement, une commande de synchronisation.

3. Procédé (400) selon la revendication 1, dans lequel l'étape de transmission (406) est exécutée, par l'unité de commande (101), en fonction d'un profil du programme d'entraînement choisi par l'utilisateur entraîneur, l'étape de transmission (406) étant exécutée, par l'unité de commande (101), pour doter la pluralité d'appareils d'exercice (500) de l'instruction de commande du fonctionnement de chaque appareil d'exercice (200), au moins au nombre de une, telle que la modification d'un paramètre de l'appareil d'exercice (200) qui peut être commandé pendant le fonctionnement.

4. Procédé (400) selon l'une quelconque des revendications précédentes, comprenant en outre, entre l'étape consistant à charger (403), par l'unité de commande (101), un programme d'entraînement à exécuter pour les utilisateurs de la classe d'entraînement et l'étape consistant à transmettre (404), par l'unité de commande (101), une commande de début d'entraînement à la pluralité d'appareils d'exercice (500), une étape consistant à transmettre (408), par l'unité de commande (101), le programme d'entraînement choisi par l'utilisateur entraîneur à la pluralité d'appareils d'exercice (500), au moyen du réseau de communication de données (NTW).

5. Procédé (400) selon la revendication 4, comprenant en outre une étape consistant à exécuter (409), par chaque appareil d'exercice (200) de la pluralité d'appareils d'exercice (500), le programme d'entraînement reçu en provenance de l'unité de commande (101), l'instruction de commande du fonctionnement de chaque appareil d'exercice (200), au moins au nombre de une, telle que la modification d'un paramètre de l'appareil d'exercice (200) qui peut être commandé pendant le fonctionnement, étant exécutée de manière autonome par chaque appareil d'exercice (200).

6. Procédé (400) selon la revendication 5, dans lequel chaque appareil d'exercice (200) de la pluralité d'appareils d'exercice (500) est un tapis roulant, ledit paramètre d'appareil d'exercice qui peut être commandé pendant le fonctionnement étant l'inclinaison du tapis roulant, l'étape de commande (407) étant exécutée par commande d'un dispositif d'actionnement (206) dont chaque appareil d'exercice est pourvu, configuré pour recevoir une commande de modification d'inclinaison de tapis roulant en provenance d'une unité de traitement de données respective (201) de l'appareil d'exercice (200).

7. Procédé (400) selon l'une quelconque des revendications précédentes, dans lequel l'étape consistant à transmettre (406) l'instruction de commande du fonctionnement d'un appareil d'exercice (200), au moins au nombre de une, est exécutée, par l'unité de commande (101), en réponse à la réception d'une demande respective de réception d'une instruction de commande du fonctionnement d'un appareil d'exercice (200) et de données d'entraînement de chaque utilisateur sur un appareil d'exercice respectif (200), enregistrées par l'appareil d'exercice (200) pendant son fonctionnement, transmises périodiquement par chaque appareil d'exercice (200) de la pluralité d'appareils d'exercice (500).

8. Procédé (400) selon l'une quelconque des revendications précédentes, comprenant en outre une étape consistant à fournir, par l'unité de commande (101), une information représentative du temps d'entraînement pendant l'exécution du programme d'entraînement à la pluralité d'appareils d'exercice (500), au moyen du réseau de communication de données (NTW).

9. Procédé (400) selon l'une quelconque des revendications précédentes, comprenant en outre une étape consistant à présenter à l'utilisateur entraîneur, par l'unité de commande (101), au moyen d'un module d'affichage respectif (105) dont l'unité de commande est pourvue (101), une liste des utilisateurs participant au programme d'entraînement, dans lequel les données d'identification d'utilisateur et une indication représentant le fait qu'un utilisateur peut suivre ou non un profil d'un premier paramètre de l'appareil d'exercice qui peut être commandé pendant le fonctionnement, selon le programme d'entraînement chargé, sont associées à chaque utilisateur, ladite étape de présentation comprenant les étapes consistant à :
- recevoir une valeur dudit premier paramètre de l'appareil d'exercice (200) qui peut être commandé pendant son fonctionnement, pendant l'entraînement d'un utilisateur sur l'appareil d'exercice (200) ;
- comparer une telle valeur reçue à la valeur dudit premier paramètre de l'appareil d'exercice (200) qui peut être commandé pendant son fonctionnement envisagée par le programme d'entraînement afin de déterminer un écart ;
- si un tel écart est différent de zéro, déplacer une ligne à l'intérieur de la pluralité de lignes (PR) correspondant à un tel utilisateur vers la première place de la pluralité de lignes (PR), ajouter un symbole graphique (SG) sur le côté d'au moins l'un ou d'une combinaison de : un numéro séquentiel (01), un nom réel respectif (RN1) et un pseudonyme respectif (NK1) d'un tel utilisateur.

10. Procédé (400) selon l'une quelconque des revendications précédentes, comprenant en outre une étape consistant à transmettre, par chaque appareil d'exercice (200) de la pluralité d'appareils d'exercice (500), à l'unité de commande (101), au moyen du réseau de communication de données (NTW), pendant l'exécution du programme d'entraînement, une demande d'instructions de commande du fonctionnement de l'appareil d'exercice (200) et de données d'entraînement de l'utilisateur sur l'appareil d'exercice (200), telle qu'au moins l'une ou une combinaison de :
- la valeur d'un premier paramètre de l'appareil d'exercice qui peut être commandé pendant le fonctionnement ;
- la valeur d'un second paramètre de l'appareil d'exercice qui peut être commandé pendant le fonctionnement ;
- des données représentatives de la condition physique de l'utilisateur pendant l'entraînement ;
- des données d'identification d'utilisateur.

11. Procédé (400) selon l'une quelconque des revendications précédentes, comprenant en outre une étape consistant à modifier manuellement, à tout moment pendant l'exécution du programme d'entraînement, suite à une commande reçue en provenance de l'utilisateur par l'intermédiaire d'une interface de commande (203) dont un appareil d'exercice (200) de la pluralité d'appareils d'exercice (500) est pourvu, une valeur d'un premier paramètre de l'appareil d'exercice qui peut être commandé pendant le fonctionnement ou d'un second paramètre de l'appareil d'exercice qui peut être commandé pendant le fonctionnement si l'utilisateur ne peut pas maintenir la valeur du premier paramètre de l'appareil d'exercice qui peut être commandé pendant le fonctionnement ou la valeur du second paramètre de l'appareil d'exercice qui peut être commandé pendant le fonctionnement, définies automatiquement pendant le programme d'entraînement choisi par l'utilisateur entraîneur.

12. Procédé (400) selon l'une quelconque des revendications précédentes, comprenant une étape consistant à fournir à l'utilisateur, par l'appareil d'exercice respectif (200) au moyen d'une unité d'affichage (204) dont l'appareil d'exercice (200) est pourvu, une information représentative d'une ou de plusieurs valeurs préférées d'un second paramètre de l'appareil d'exercice (200) qui peut être commandé pendant son fonctionnement, déterminées sur la base de la valeur du premier paramètre de l'appareil d'exercice (200) qui peut être commandé pendant son fonctionnement.

13. Procédé (400) selon l'une quelconque des revendications précédentes, comprenant en outre les étapes consistant à :
- transmettre (410), par l'unité de commande (101), une commande de fin d'entraînement à la pluralité d'appareils d'exercice (500), au moyen du réseau de communication de données (NTW) ;
- mettre fin (411), par la pluralité d'appareils d'exercice (500), à l'entraînement suite à la réception de la commande de fin d'entraînement.

14. Procédé (400) selon l'une quelconque des revendications précédentes, comprenant une étape consistant à fournir un processeur électronique distant (300) fonctionnellement connecté à l'unité de commande (101) et à la pluralité d'appareils d'exercice (200), le procédé (400), après la transmission d'une commande de fin d'entraînement, à la fin de l'entraînement, comprenant les étapes consistant à:
- stocker, par l'unité de commande (101), un résumé de la classe d'entraînement dans un module de mémoire respectif (103) ;
- transmettre, par l'unité de commande (101), au processeur électronique distant (300), le résumé de la classe d'entraînement afin de le stocker dans une unité de commande (302) du processeur électronique distant (300) dans un compte personnel d'utilisateur entraîneur.

15. Procédé (400) selon la revendication 14, comprenant, suite à la réception de la commande de fin d'entraînement, une étape consistant à transmettre, par chaque appareil d'exercice (200) de la pluralité d'appareils d'exercice (500), au processeur électronique distant (300), des résultats d'entraînement obtenus par l'utilisateur sur l'appareil d'exercice (200), afin de les stocker dans l'unité de mémoire (302) du processeur électronique distant (300), dans un compte d'utilisateur.

16. Système (100) permettant de gérer un entraînement d'utilisateurs sur une pluralité d'appareils d'exercice (200), comprenant :
- une unité de commande (101) d'une classe d'entraînement d'utilisateurs sur une pluralité d'appareils d'exercice (500), l'unité de commande (101) étant accessible par un utilisateur entraîneur chargé de gérer une classe d'entraînement sur une pluralité d'appareils d'exercice (500) ;
- une pluralité d'appareils d'exercice (500) fonctionnellement connectés à l'unité de commande (101) au moyen d'un réseau de communication de données (NTW) ;
le système (100) étant configuré pour exécuter le procédé (400) permettant de gérer un entraînement d'utilisateurs sur une pluralité d'appareils d'exercice (500) selon l'une quelconque des revendications précédentes 1 à 13.

17. Système (100) selon la revendication 16, comprenant en outre un processeur électronique distant (300) fonctionnellement connecté à l'unité de commande (101) et à la pluralité d'appareils d'exercice (500), le système (100) étant configuré pour exécuter le procédé (400) permettant de gérer un entraînement d'utilisateurs sur une pluralité d'appareils d'exercice (500) selon l'une quelconque des revendications précédentes 14 ou 15.
